# EUROPEAN PATENT APPLICATION

(11) **EP 2 581 101 A1**
(43) Date of publication of application: **17.04.2013**
(21) Application number: 12188043.9
(22) Date of filing: 10.10.2012
(51) Int. Cl.: A61M 5/31, A61M 5/34

(54) **Improvements in syringes**

(30) Priority: 10.10.2011 GB 201117434; 09.10.2012 US 201213647955
(71) Applicant: Shams, Iden, Richmond Surrey TW10 5EA (GB)
(72) Inventor: Shams, Iden, Richmond Surrey TW10 5EA (GB)
(74) Representative: ip21 Ltd

(57) **Abstract**

A syringe tip (1) for a syringe and the like in which the bore (2) of the tip tapers so as to increase in diameter from the end of the tip (4) adjacent the barrel of a syringe towards the distal end (3) of the tip; and the outside diameter of said tip (1) tapers so as to decrease in diameter from the proximal end of the tip (adjacent the barrel of said syringe) towards the distal end of the tip (3).

## Description

### Field of the Invention

The invention relates to syringes, and particular to connection systems for syringes, and especially for enteral syringes.

### Background and Prior Art Known to the Applicant

For many years, a 6% taper has been used on medical connectors to connect syringes with needles and other fluid-transporting devices such as intravenous catheters, valves and filters. This 6% taper is commonly described as a "Luer" connection, named after the century German medical instrument maker Hermann Wülfing Luer, the 6% taper having previously been used successfully for tapered glass stoppers for bottles. A typical Luer connection uses an externally-tapered male connection for syringes, and a corresponding 6% internal taper for needles. The Luer standard (and its specification is enshrined in international standard ISO 594:1986 "Conical fittings with a 6 % (Luer) taper for syringes, needles and certain other medical equipment") has been so successful that it became the connection of choice for all kinds of delivery systems including enteral, intravenous and intrathecal.

However, it became apparent that this standard allowed inadvertent misconnection of drug-filled syringes and other fluid-containing devices causing misadministration of medicaments into the wrong part of the body, sometimes with fatal consequences.

One reaction to this has been the development of separate "standards" for enteral connectors - i.e. for connectors destined to be used on syringes, tubes and other medical devices to deliver drugs, nutritional formulations and other fluids into the gastrointestinal tract.

One such system, described in US Patent Application US2007/0076401 in the name of Vygon, typically uses a 5% taper connection. In the Vygon system, syringes are usually provided with female (internal) tapered connections (as opposed to the male 6% taper in the Luer system); a smaller diameter than the standard Luer diameter is also typically used.

In another system, sold by Fresenius Kabi, a larger 2% taper connection is used, with syringes being provided with an external taper - i.e. a "male" fitting.

In yet further systems, a so-called "catheter tip connector" is used, having a yet larger tapered connection, but again using an external taper - a "male" fitting - on syringe elements.

Whilst these systems have been successful in preventing misadministration of enteral formulations by other routes (and vice-versa), the presence of different enteral systems can cause problems in health-care settings where more than one of the different enteral standards are used. This can occur even in the same hospital where, for example, one system might be used on a paediatric ward, and another system on general wards. This can lead to situations where a patient is transferred from one ward to another, and appropriately-connecting syringes are unavailable to connect with medical devices already attached to a patient.

It is amongst the objects of the present invention to attempt a solution to this and other problems.

At the time of writing, the applicant acknowledges the following prior art documents: WO2009/144583A1 BECTON, WO2010/14019A1 BECTON, US5609584A GETTIG, US4596561A MEYER, and WO8805668A1 MEYER

### Summary of the Invention

Accordingly, the invention provides a syringe tip for a syringe and the like in which the bore of said tip tapers so as to increase in diameter from the proximal end of the tip (adjacent the barrel of said syringe) towards the distal end of the tip; and the outside diameter of said tip tapers so as to decrease in diameter from the proximal end of the tip (adjacent the barrel of said syringe) towards the distal end of the tip.

Preferably, a syringe tip for a syringe and the like where the syringe tip extends from the barrel of a syringe; said syringe tip comprises an internal bore and an outside diameter; said internal bore incorporating a taper at the distal most portion of the tip; said taper continuously increasing in diameter from the proximal end of the tip which is adjacent the barrel of the syringe towards the distal end of the tip; whereby said taper facilitates a liquid-tight connection to any appropriately mating connector; and the outside diameter of the tip tapers so as to decrease in diameter in the distal most portion of the tip towards the distal end of the tip; whereby said taper facilitates a liquid tight connection to any appropriately mating connector.

Preferably the external taper of said tip is not 6%.

Preferably also, the taper of the bore of said tip is not 6%.

In either case, it is preferable that the external taper of said tip is between 1% and 4%.

It is also preferable that the taper of the bore of said tip is less than 6%, and preferably between 3% and 5.5%.

In any aspect of the invention the syringe tip further comprises an externally-projecting locking portion on the outside of said tip.

In a further aspect, the invention provides a syringe wherein a portion of the outside surface of the syringe barrel adjacent the syringe tip is tapered, having an outside diameter that decreases towards the syringe tip, thereby being capable of forming a fluid-tight connection with a correspondingly internally tapered connector.

Preferably said tapered portion of the barrel is graduated.

More preferably said syringe further comprises a syringe tip as described above.

Also included within the scope of the invention is a syringe tip or a syringe substantially as described herein with reference to any appropriate combination of the accompanying drawings.

Also included within the scope of the invention is a syringe comprising a syringe barrel and a syringe tip as described herein.

In a further independent aspect, the invention provides a syringe tip and connector assembly comprising, a syringe tip for a syringe and the like where the syringe tip extends from the barrel of a syringe; said syringe tip comprising an internal bore and an outside diameter; said internal bore incorporating a taper at the distal most portion of the tip; said taper continuously increasing in diameter from the proximal end of the tip which is adjacent the barrel of the syringe towards the distal end of the tip; and the outside diameter of the tip tapers so as to decrease in diameter in the distal most portion of the tip towards the distal end of the tip; and a connector body with at least one of an outer diameter to facilitate a liquid-tight connection with said internal bore of said syringe tip; and an inner diameter configured to facilitate a liquid-tight connection with said external diameter of said syringe tip.

In a subsidiary aspect, the connector body comprises an outer diameter and an inner diameter both configured to facilitate a liquid-tight connection with both said outside diameter and said internal bore of said tip.

Many of the embodiments of the invention improve connectability, versatility and in particular the liquid-tightness property of syringe tips.

### Brief Description of the Drawings

The invention will be described with reference to the accompanying drawings, in which:
Figures 1 and 2 are cross-sections of syringe tips of the invention;
Figure 3 is a perspective view of a syringe tip and syringe of the invention;
Figure 4 is a cut-away perspective view of a syringe tip and syringe of the invention;
Figure 5 is a perspective view of a syringe of the invention; and
Figure 6 is a cut-away perspective view of a syringe of the invention.
Figures 7, 8 and 9 are cross-sections of assemblies of the invention.

### Description of Preferred Embodiments

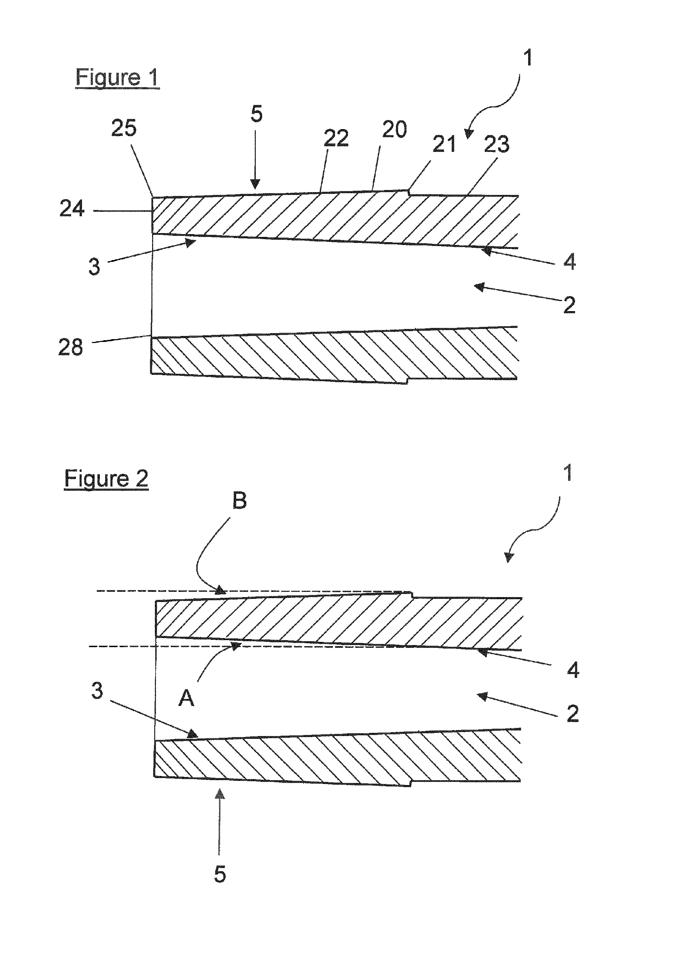

Figures 1 and 2 show, in cross-sectional view, a syringe tip, generally indicated by 1, according to the invention. In this tip, the bore 2 of the tip 1 is tapered, such that the internal diameter of the bore adjacent the discharge end of the tip 3 is larger than the internal diameter at the end of the tip 4 adjacent the barrel of the syringe. The internal taper, indicated by angle A in Figure 2, is arranged to be at an angle that is incompatible with a male Luer connector, i.e. it is not a 6% taper. Preferably, the taper is configured to be less than 6%. The taper is preferably between 3% and 5.5%. This prevents interconnectivity with Luer fittings destined for use with medicaments not intended to be delivered enterally, and vice-versa. Most preferably, a 5% internal taper is used. In this way, the internal bore of the syringe tip will fluidly mate with a Vygon connector as described above.

The external surface 5 of the syringe tip 1 is also provided with a taper such that the external diameter of syringe tip at the discharge end is smaller than the diameter at the end of the tip adjacent a syringe barrel. The external taper, indicated by B in figure 2, is again configured not to fluidly mate with a Luer connector, i.e. it is not a 6% taper. In preferred embodiments, the external taper B is between 1% and 4%, and preferably the taper is 2%. A 2% taper is equivalent to a taper of 3.43° (i.e. 1.74° for each side). In this way, the external surface will mate with Fresenius fittings as described above.

Whilst it is especially and preferably envisaged that such syringe tips will form part of a syringe, it is also envisaged that the tips could be used as a general connector for enteral lines, for example as part of enteral "giving sets", being fluidly-connected e.g. to the end of a tube. In this case, references to "adjacent the syringe barrel" and like terms may be construed as "adjacent the tube", and most generally meaning upstream of the fluid connection.

At figure 1 it can clearly be seen that the bore 2 of the tip 1 comprises a continuous taper from end 24 inwards. This allows for a continuous, single, uninterrupted contact between the bore 3 and a suitably disposed connector. This contact forms an internal fluid tight (water tight) attachment between a connector and the tip of the syringe. The external surface 5 of the syringe tip 1 comprises a continuous taper 20 along the majority of its length for a similar continuous, uninterrupted contact with a suitably disposed connector (not shown). This contact facilitates an external fluid tight attachment between a connector and the external surface of the syringe tip. However, in this preferred embodiment there is also shown a step 21 which splits the taper of the external surface into a first side 22 and a second side 23 - thus along the whole of its length the taper of the external surface 5 of the syringe tip 1 is discontinuous although it is the case that each of first side 22 and second side 23 is linear (like the taper of bore 3) in its taper. Indeed in particularly preferred embodiments, the taper of the first side 22 and the taper of the second side 23 run along notionally parallel linear paths, sharing as they do the same angle. In other words, the first portion of the tip both externally and internally, starting from the distal most extremity 24 of the syringe tip comprises a continuous taper which facilitates in a first mode of use an external liquid tight attachment to a connector and in a second mode of use an internal liquid tight attachment to a connector.

Whilst the syringe tip 1 as illustrated comprises an end 24 wherein the end comprises a squared off outer edge 25 and a squared off inner edge 28, in an alternative embodiment the edges of the distal or discharge end 3 of the tip may be rounded or chamfered.

A further advantageous embodiment of the invention would incorporate an external surface 5 which incorporates a taper which continuously increases towards the barrel of the syringe. In this configuration, there would be no step internally or externally - thus providing an uninterrupted smooth surface onto which one or a plurality of connectors may fit and form a liquid-tight attachment.

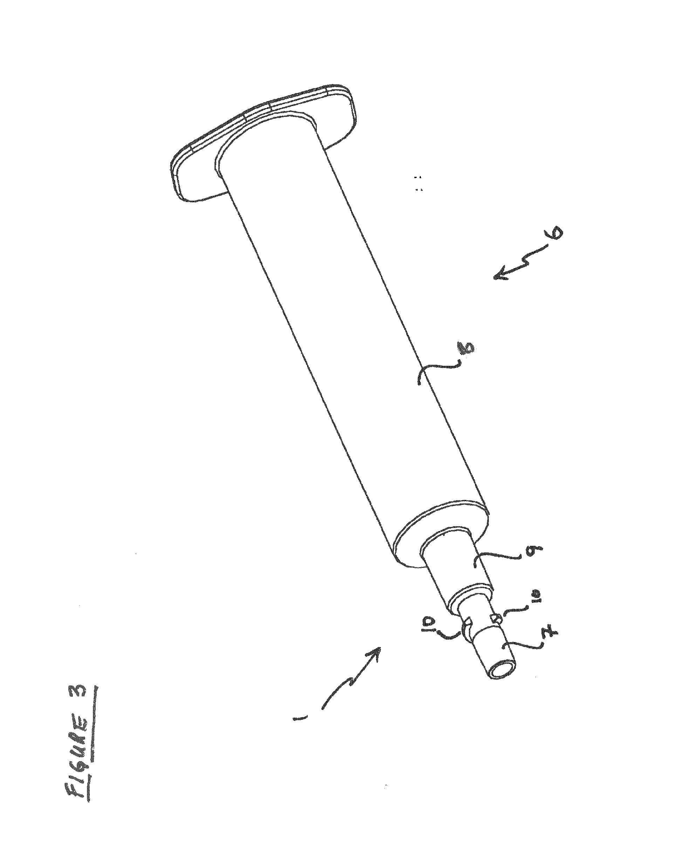

Figure 3 illustrates, in perspective view, a further syringe tip, generally indicated by 1, forming part of a syringe, generally indicated by 6. In this embodiment, the syringe tip 1 has a first externally tapered portion 7 at the end distal from the syringe barrel 8. The configuration of this portion is as described above for the syringe tip of Figures 1 and 2. The bore of this syringe tip portion 7 is also internally tapered, again as described above for the syringe tip of Figures 1 and 2.

The syringe tip also has a second externally tapered portion 9 located adjacent the syringe barrel 8. This second tapered portion 9 has a taper such that the external diameter of the portion decreases in a direction away from the end proximal to the barrel 8 and towards the discharge end of the tip 1. The taper in this portion is configured to be that of a catheter taper, i.e. preferably a taper of 2°.

In this embodiment, outwardly-projecting lugs 10 are provided between the two externally tapered portions 7 and 9. The lugs are preferably configured to form part of a helix. The lugs may be used to lock the end of the syringe tip to a connector having a corresponding internal thread arrangement, such as is found in the Vygon connector described above.

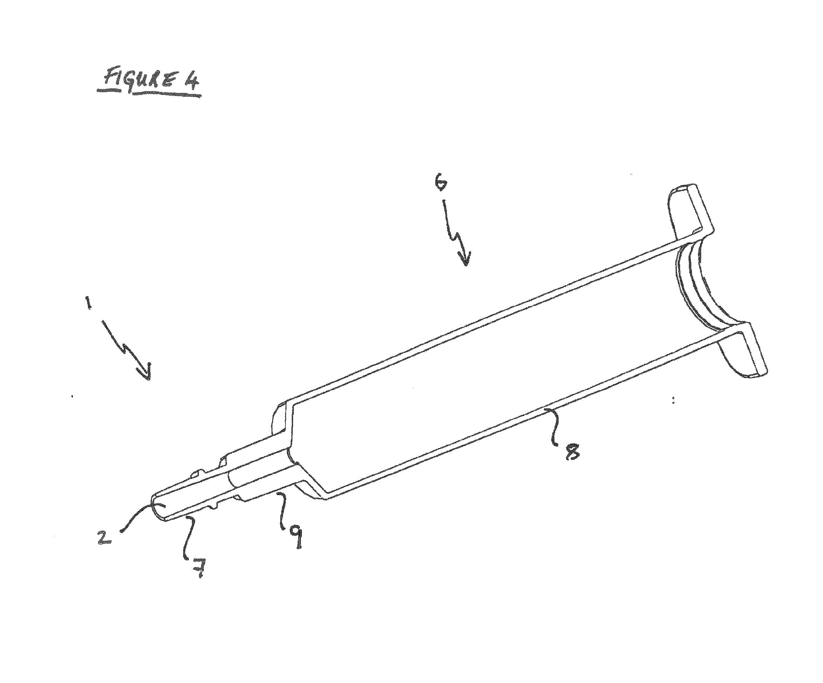

Figure 4 illustrates in cut-away perspective view, the syringe tip and syringe of Figure 3. The cutaway view illustrates that the internal bore 2 of the first tapered portion 7 is also tapered, as described for the tip illustrated in Figures 1 and 2.

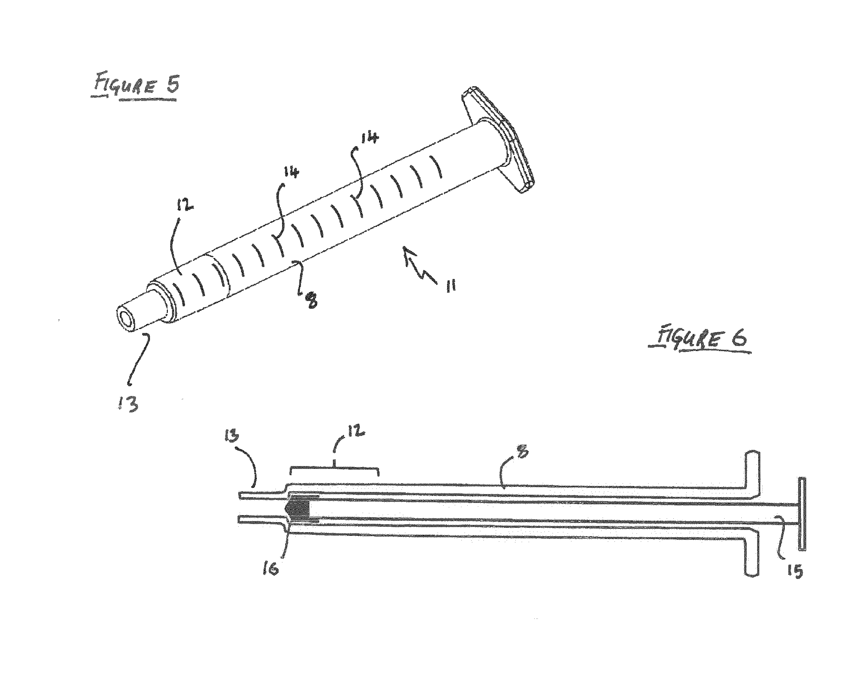

Figure 5 illustrates a further embodiment of an aspect of the invention in perspective view. This embodiment comprises a syringe, generally indicated by 11 in which a portion 12 of the external surface of the syringe barrel 8 is provided with a taper, in which the outside diameter of the barrel 8 decreases towards the discharge end of the syringe. Preferably the taper is 2°, such that it can function as a catheter tip connector.

At the end of the syringe is a syringe tip 13. In preferred embodiments, this tip is provided with an external, and preferably an internal taper, as described for the embodiment of Figures 1 and 2.

The external body of the barrel 8 is also marked with graduations 14 to indicate volume. This configuration of syringe is particularly suited to small volume syringes, and particularly those having a volume of about 1ml.

Figure 6 shows the syringe of Figure 5 in cross-sectional view, also including a plunger 15, slideably sealed within the barrel 8 of the syringe 11. It can be seen that the sealing end of the plunger 16 extends into the portion of the barrel 12 that is tapered, i.e. it extends completely into the connector portion. In this way, dead space in the connector is effectively eliminated.

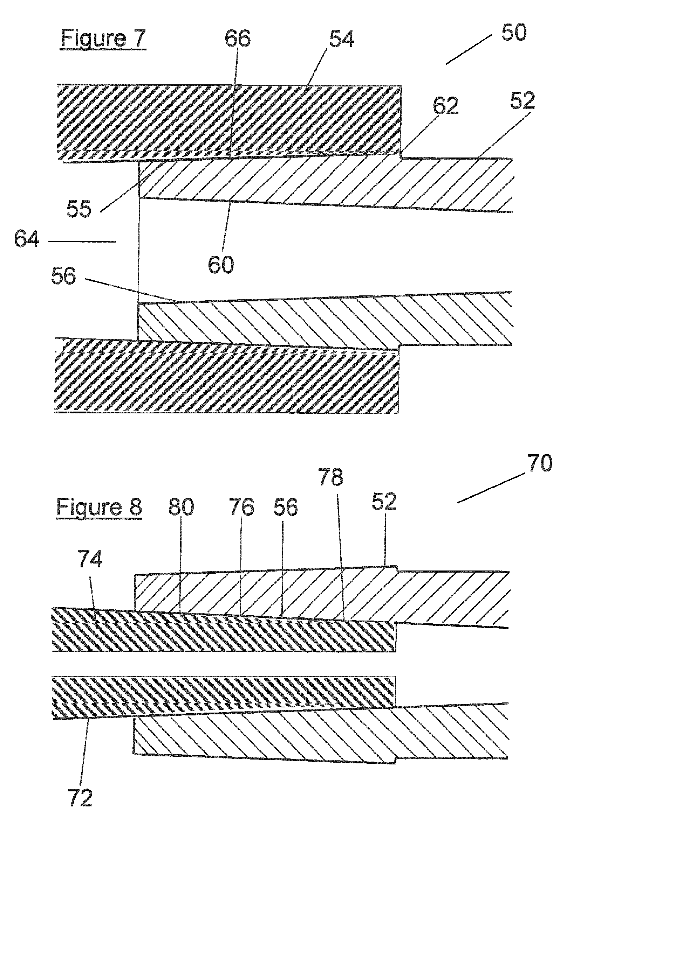

At Figure 7, an assembly 50 is shown. The assembly comprises a syringe tip 52 and a connector 54. The syringe tip 52 comprises an internal bore 56 with the taper of the type described in the foregoing paragraphs. The syringe tip also comprises an outer diameter 55 with a taper also so described in the foregoing paragraphs. The taper of the internal bore 56 comprises a first syringe tip contact surface 60, whilst the outer diameter 55 comprises a second syringe tip contact surface 62. In this assembly, the connector 54 is so sized that the forward most portion of the syringe tip 52 fits inside it thereby forming a liquid tight (eg. water tight) connection. The connector 54 comprises a tube with a taper 66 which decreases in diameter from its distal extremity inwards. The syringe 52 and the connector 54 are shown mated with one another and as such the taper 66 on the inner diameter of the tube which comprises a first tube contact surface is at a complementary angle to that of the outer diameter 55 of the syringe tip 52, mates with it and forms a continuous, linear, uninterrupted, liquid tight connection

At Figure 8, a second assembly 70 is shown - whilst syringe tip 52 remains essentially the same, the connector 72 here fits inside the bore 56 of the syringe tip 52. The connector 72 comprising a tube 74 and having an outer diameter 76 which itself has a taper, the taper decreasing the outer diameter 76 of the tube 74 prior to the termination of the tube 74 at its tip. The taper of the outer diameter 76 of the tube 74 matches the taper of the bore 56 of the syringe tip 52. The taper of the outer diameter 76 of the tube 74 defines a second tube contact surface 78 which mates with and forms a continuous, linear, uninterrupted liquid tight connection with first syringe tip contact surface 56.

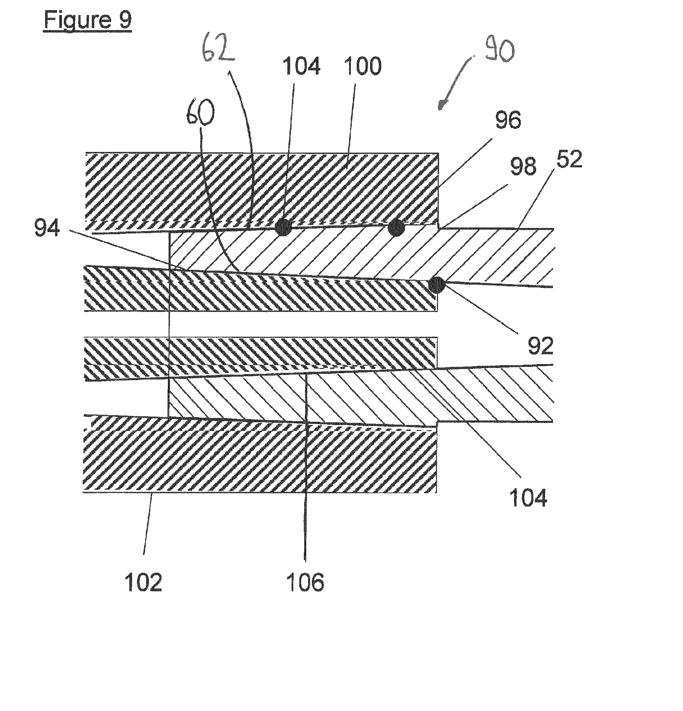

At figure 9, a third assembly 90 is shown, wherein the syringe tip 52 comprises an inner bore with a taper comprising a first syringe tip contact surface 94 and an outer diameter 96, comprising a second syringe contact surface 98. The syringe tip mates with connector 100 comprising a bifurcated tube 102 which fits on both the inside and outside of syringe tip 92, having a first tube contact surface 104 and a second tube contact surface 106, each of those contact surfaces 104, 106 having a taper corresponding to the mating contact surfaces 60, 62 of the syringe tip. In this configuration, there is provided a succession of an internal fluid tight connection and an external fluid tight connection.

In a preferred embodiment of any of the figures 1- 9, the syringe tip 1 may be an integrally formed part of the syringe 6.

## Claims

1. A syringe tip for a syringe and the like where the syringe tip extends from the barrel of a syringe; said syringe tip comprising an internal bore and an outside diameter; said internal bore incorporating a taper at the distal most portion of the tip; said taper continuously increasing in diameter from the proximal end of the tip which is adjacent the barrel of the syringe towards the distal end of the tip; whereby said taper facilitates a liquid-tight connection to any appropriately mating connector; and the outside diameter of the tip tapers so as to decrease in diameter in the distal most portion of the tip towards the distal end of the tip; whereby said taper facilitates a liquid tight connection to any appropriately mating connector.

2. A syringe tip according to claim 1, wherein the external taper of said tip is not 6%.

3. A syringe tip according to either claim 1 or claim 2, wherein the taper of the bore of said tip is not 6%.

4. A syringe tip according to any of the preceding claims, wherein the external taper of said tip is between 1% and 4%.

5. A syringe tip according to any of the preceding claims, wherein the taper of the bore of said tip is less than 6%.

6. A syringe tip according to any of the preceding claims, wherein the taper of the bore of said tip is between 3% and 5.5%.

7. A syringe tip according to any of the preceding claims, further comprising an externally-projecting locking portion on the outside of said tip.

8. A syringe comprising a barrel and a syringe tip with an internal bore and an outside diameter; said internal bore incorporating a taper at the distal most portion of the tip; said taper continuously increasing in diameter from the proximal end of the tip which is adjacent the barrel of the syringe towards the distal end of the tip; whereby said taper facilitates a liquid-tight connection to any appropriately mating connector; and the outside diameter of the tip tapers so as to decrease in diameter in the distal most portion of the tip towards the distal end of the tip; whereby said taper facilitates a liquid-tight connection to any appropriately mating connector.

9. A syringe according to claim 8, wherein said tapered portion of the barrel is graduated.

10. A syringe tip and connector assembly comprising,
a syringe tip for a syringe and the like where the syringe tip extends from the barrel of a syringe; said syringe tip comprising an internal bore and an outside diameter; said internal bore incorporating a taper at the distal most portion of the tip; said taper continuously increasing in diameter from the proximal end of the tip which is adjacent the barrel of the syringe towards the distal end of the tip; and the outside diameter of the tip tapers so as to decrease in diameter in the distal most portion of the tip towards the distal end of the tip;
and
a connector body with at least one of an outer diameter to facilitate a liquid-tight connection with said internal bore of said syringe tip; and an inner diameter configured to facilitate a liquid-tight connection with said external diameter of said syringe tip.

11. A syringe tip and connector assembly according to claim 10, wherein said connector body comprises an outer diameter and an inner diameter both configured to facilitate a liquid-tight connection with both said outside diameter and said internal bore of said tip.
